# EUROPÄISCHE PATENTANMELDUNG

(11) **EP 2 574 577 A2**
(43) Veröffentlichungstag der Anmeldung: **03.04.2013**
(21) Anmeldenummer: 12186363.3
(22) Anmeldetag: 27.09.2012
(51) Int. Cl.: B65H 23/32

(54) **Verfahren zur Bearbeitung einer Materialbahn, insbesondere zur Herstellung von Hygieneprodukten**

(30) Priorität: 29.09.2011 DE 202011106176 U
(71) Anmelder: Bikoma GmbH Spezialmaschinen, 56727 Mayen (DE)
(72) Erfinder: Göbel, bernd, 56761 Gamlen (DE)
(74) Vertreter: Lemcke, Brommer & Partner

(57) **Zusammenfassung**

Vorgeschlagen wird ein Verfahren zum Bearbeiten einer Materialbahn (2), insbesondere zur Herstellung von Hygieneprociukten, wie Einlagen, Windeln oder dergleichen, bei dem an oder auf der Materialbahn weitere Materialien angebracht oder aufgebracht werden, vorzugsweise aufgeklebt, aufgepresst, aufgeriegelt oder dergleichen, welches sich dadurch auszeichnet, dass die Materialbahn (2) a) mittels wenigstens einer ersten Bearbeitungseinheit (5.9) mit einer ersten Bearbeitungsrichtung, vorzugsweise von oben in Richtung der Schwerebeschleunigung, bearbeitet wird, wobei eine erste Materialapplikation (MA1) erfolgt; b) durch wenigstens eine Wendeeinrichtung (1) geführt wird, um die Materialbahn (2) in der Wendeeinrichtung (1) im Wesentlichen um 180° zu wenden, ohne eine Förderrichtung der Materialbahn (2) zu verändern; c) mittels wenigstens einer zweiten Bearbeitungseinheit (5.2) mit einer zweiten Bearbeitungsrichtung, vorzugsweise von oben in Richtung der Schwerebeschleunigung, bearbeitet wird, wobei eine zweite Materialapplikation (MA2) erfolgt; wobei die erste Bearbeitungsrichtung und die zweite Bearbeitungsrichtung im Wesentlichen gleich orientiert sind.

## Beschreibung

Die Erfindung betrifft ein Verfahren nach dem Oberbegriff des Anspruchs 1 zum Bearbeiten einer Materialbahn, insbesondere zur Herstellung von Hygieneprodukten.

Die Erfindung betrifft außerdem eine Wendeeinrichtung nach dem Oberbegriff des Anspruchs 5 für eine Materialbahn, mit wenigstens einem länglichen Wendemittel mit zumindest abschnittweise gewölbter Mantelfläche, über dessen Mantelfläche die Materialbahn geführt ist, um eine Transport- oder Förderrichtung der Materialbahn umzulenken, sowie eine Materialbahnverarbeitungsmaschine nach dem Oberbegriff des Anspruchs 13.

Wendeeinrichtungen der genannten Art sind dem Fachmann beispielsweise in Form sogenannter Wendestangen bekannt. Sie dienen im Maschinenbau dazu, eine geförderte Materialbahn, wie sie beispielsweise zur Herstellung von Hygieneprodukten in einer entsprechenden Materialbahnverarbeitungsmaschine verwendet wird, in ihrer Transport- oder Förderrichtung umzulenken, um sie beispielsweise bestimmten Bearbeitungsbereichen oder Bearbeitungseinheiten innerhalb der Maschine zuzuführen.

Insbesondere bei der Herstellung von Hygieneprodukten, wie Slipeinlagen, Binden, Inkontinenzprodukte und Babywindeln, ist es üblich, dass diese Produkte bzw. die Materialbahn, aus welcher die genannten Produkte hergestellt werden, auf vakuumbeaufschlagten Transportbändern in der Maschine transportiert werden und dass beim Durchlauf der Materialbahn verschiedene Bearbeitungsschritte durchgeführt werden. Diese Bearbeitungsschritte umfassen beispielsweise das Applizieren weiterer Materialien - getaktet oder durchlaufend - auf die Materialbahn, so dass ein sogenannter Produktionsstrang resultiert, wie auch die Bearbeitungsschritte des Schneidens, Prägens oder Schweißens. Insbesondere die vorstehend aufgezählten Hygieneprodukte weisen regelmäßig eine körperzugewandte Seite auf, die auch als Topsheet bezeichnet wird und die in der Regel aus einem durchlässigen (Nonwoven-)Material besteht. Außerdem umfassen die genannten Produkte eine körperabgewandte Seite, die auch als Backsheet bezeichnet wird und flüssigkeitsdicht ausgebildet ist. Zwischen diesen Materialien liegt ein saugfähiger Kern, der meist aus gemahlener Zellulose (Flocke) gebildet ist. Entsprechend den genannten Produkten ist auch die zu deren Herstellung verwendete Materialbahn bzw. der Produktionsstrang mehrlagig aufgebaut.

Kleinere Hygieneprodukte, wie Slipeinlagen, Binden oder Produkte für leichte Inkontinenz, werden dabei in der Regel derart produziert, dass das luftdurchlässige Topsheet zum Transportband orientiert ist. Es hat den Vorteil, dass die Luft im Produkt während des Transportvorgangs aus dem Produkt herausgesaugt wird, wodurch das Produkt "steifer" wird und die Bearbeitungsschritte mit größerer Präzision durchgeführt werden können. Dies ist insbesondere auch beim Falten des Produktes von Vorteil. Des Weiteren werden bei den genannten kleineren Produkten die erforderlichen Bearbeitungsschritte in der Regel am Backsheet, das heißt auf der dann frei nach oben liegenden Seite durchgeführt, was aus prozesstechnischer Sicht einfacher zu handhaben ist.

Dagegen erfolgt die Bearbeitung von größeren Produkten, wie Windeln (für Kinder oder Erwachsene) genau umgekehrt, das heißt am Topsheet, weswegen solche Produkte in der Regel mit dem Backsheet zum Transportband produziert werden. Je nach Produktaufbau kann es jedoch auch erforderlich sein, zur Herstellung eines Produkts Bearbeitungsschritte an beiden Seiten des Produkts bzw. der Materialbahn vorzunehmen.

Der Erfindung liegt deshalb die Aufgabe zugrunde, ein Verfahren zur Materialbahnbearbeitung für die Herstellung von Hygieneprodukten anzugeben, dass sich in maschineller Hinsicht einfacher, ökonomischer und schneller durchführen lässt. Zudem besteht die Aufgabe, eine Wendeeinrichtung für eine Materialbahn anzugeben, mit der es möglich ist, mit nur einer Bearbeitungsrichtung, beispielsweise von oberhalb eines Transportbandes, sowohl auf die Oberseite als auch auf die Unterseite der Materialbahn einzuwirken, wobei aus Gründen der Prozessergonomie und des erforderlichen Bauraums dafür Sorge zu tragen ist, dass die Förderrichtung der Materialbahn - bis auf einen tolerierbaren Ebenenversatz - nicht verändert wird. Außerdem soll eine Materialbahnverarbeitungsmaschine angegeben werden, welche die vorstehend beschriebene Bearbeitung einer Materialbahn ermöglicht.

Die Erfindung löst diese Aufgabe mittels eines Verfahrens gemäß Anspruch 1, mittels einer Wendeeinrichtung mit den Merkmalen des Anspruchs 5 sowie mittels einer Materialbahnverarbeitungsmaschine mit den Merkmalen des Anspruchs 13. Vorteilhafte Weiterbildungen der erfindungsgemäßen Wendeeinrichtung sind Gegenstand von Unteransprüchen, deren Wortlaut hiermit durch ausdrückliche Bezugnahme in die Beschreibung aufgenommen wird, um Textwiederholungen zu vermeiden.

Erfindungsgemäß ist ein Verfahren zum Bearbeiten einer Materialbahn, insbesondere zur Herstellung von Hygieneprodukten, wie Einlagen, Windeln oder dergleichen, bei dem an oder auf der Materialbahn weitere Materialien angebracht oder aufgebracht werden, vorzugsweise aufgeklebt, aufgepresst, aufgeriegelt oder dergleichen, dadurch gekennzeichnet, dass_die Materialbahn a) mittels wenigstens einer ersten Bearbeitungseinheit mit einer ersten Bearbeitungsrichtung, vorzugsweise von oben in Richtung der Schwerebeschleunigung, bearbeitet wird, wobei eine erste Materialapplikation erfolgt; b) durch wenigstens eine Wendeeinrichtung geführt wird, um die Materialbahn in der Wendeeinrichtung im Wesentlichen um 180° zu wenden, ohne eine Förderrichtung der Materialbahn zu verändern; c) mittels wenigstens einer zweiten Bearbeitungseinheit mit einer zweiten Bearbeitungsrichtung, vorzugsweise von oben in Richtung der Schwerebeschleunigung, bearbeitet wird, wobei eine zweite Materialapplikation erfolgt; wobei die erste Bearbeitungsrichtung und die zweite Bearbeitungsrichtung im Wesentlichen gleich orientiert sind.

Besonders vorteilhaft ist es, wenn die erste Bearbeitung und die zweite Bearbeitung im Wesentlichen von oben nach unten in Richtung der Schwerbeschleunigung erfolgt.

Wie bereits erwähnt, kann vorgesehen sein, dass die Materialbahn mehrlagig aufgebaut ist, mit einem Topsheet aus einem für Fluid durchlässigen Material und mit einem fluiddichten Backsheet, zwischen dem vorzugweise ein saugfähiger Kern angeordnet ist, und dass das Verfahren zur Herstellung von Hygieneprodukten eingesetzt wird, vorzugsweise für Binden, Windeln oder Einlagen.

Eine besondere Ausgestaltung sieht vor, dass wenigstens eine erfindungsgemäße Wendeeinrichtung im Zuge des Verfahrens zum Wenden der Materialbahn bzw. des Produktionsstrangs eingesetzt wird.

Die Materialapplikationen können lückenhaft oder lückenlos erfolgen.

Erfindungsgemäß umfasst eine Wendeeinrichtung für eine Materialbahn wenigstens ein erstes längliches Wendemittel, über dessen Mantelfläche die Materialbahn geführt ist, und wenigstens ein in Förderrichtung der Materialbahn auf das erste Wendemittel folgendes zweites längliches Wendemittel, über dessen Mantelfläche die Materialbahn ebenfalls geführt ist. Eine Längsachse des ersten Wendemittels ist dabei erfindungsgemäß unter einem Winkel von 45° zu einer Förderrichtung der Materialbahn vor einer Umlenkung durch das erste Wendemittel orientiert. Des Weiteren sind erfindungsgemäß die Längsachse des ersten Wendemittels und eine Längsachse des zweiten Wendemittels unter einem rechten Winkel zueinander orientiert und erstrecken sich außerdem jeweils parallel zu einer Ebene der Materialbahn vor der Umlenkung durch das erste Wendemittel. Die Materialbahn verläuft erfindungsgemäß zwischen dem ersten Wendemittel und dem zweiten Wendemittel derart, dass eine Zuführrichtung der Materialbahn zu dem zweiten Wendemittel antiparallel zu einer Abführrichtung der Materialbahn von dem ersten Wende mittel ausgebildet ist.

Die Materialbahn gelangt also erfindungsgemäß zunächst zu dem ersten Wendemittel, dessen Längsachse unter einem Winkel von 45° zu der Förderrichtung der Materialbahn orientiert ist. Dort erfährt die Materialbahn eine Umlenkung ihrer Förderrichtung um 90°, wobei sie gleichzeitig gewendet wird. Auf dem Weg zum zweiten Wendemittel verändert sich die Förderrichtung der Materialbahn erneut, so dass die Förderrichtung der Materialbahn beim Erreichen des zweiten Wendemittels antiparallel zu der Förderrichtung der Materialbahn beim Verlassen des ersten Wendemittels orientiert ist. Die Längsachse des zweiten Wendemittels ist relativ zu der Längsachse des ersten Wendemittels unter einem rechten Winkel angeordnet. Die Materialbahn erfährt daher an dem zweiten Wendemittel erneut eine Umlenkung ihrer Förderrichtung um 90°. Da die Längsachsen des ersten und zweiten Wendemittels außerdem parallel zueinander angeordnet sind, entspricht die Förderrichtung der Materialbahn beim Verlassen des zweiten Wendemittels - bis auf einen möglichen Ebenenversatz - gerade der Förderrichtung vor Erreichen des ersten Wendemittels. Da die Materialbahn zunächst -- wie bereits erwähnt an dem ersten Wendemittel und außerdem sowohl zwischen dem ersten und dem zweiten Wentiemittel als auch an dem zweiten Wendemittel selbst jeweils gewendet wird, erfährt die Materialbahn insgesamt also drei Wendungen, so dass sie nach Verlassen des zweiten Wendemittels gegenüber ihrer Anfangsausrichtung beim Erreichen der Wendeeinrichtung gewendet ist, wie gefordert.

Die Wendemittel können insbesondere als Wendestangen und damit zylinderförmig nach Art eines geraden Kreiszylinders ausgebildet sein. Die Erfindung ist jedoch hierauf nicht beschränkt. Auch allgemeinere Zylinderformen kommen in Betracht, ebenso wie Teilzylinder oder dergleichen, solange diese eine abschnittweise gewölbte oder gekrümmte äußere Oberfläche (Mantelfläche) aufweisen, auf der bzw. über der die Materialbahn geführt werden kann.

Um den für die Wendeeinrichtung erforderlichen Bauraum möglichst klein zu halten, sieht eine erste Weiterentwicklung der erfindungsgemäßen Wendeeinrichtung vor, dass das erste Wendemittel und das zweite Wendemittels einander in einer Richtung senkrecht zu der genannten Ebene der Materialbahn zumindest teilweise überlappen. Unter dem Begriff "genannte Ebene der Materialbahn" wird vorliegend diejenige gedachte Ebene verstanden, in welcher sich die Materialbahn vor Erreichen der Wendeeinrichtung bzw. des ersten Wendemittels bewegt.

Um den Verlauf der Materialbahn im Bereich zwischen dem ersten Wendemittel und dem zweiten Wendemittel prozesssicher und definiert auszugestalten, sieht eine äußerst bevorzugte Weiterbildung der erfindungsgemäßen Wendeeinrichtung vor, dass in Förderrichtung zwischen dem ersten Wendemittel und dem zweiten Wendemittel wenigstens noch ein drittes längliches Wendemittel angeordnet ist, über dessen zumindest abschnittweise gewölbte Mantelfläche die Materialbahn geführt ist. Dabei ist eine Längsachse des dritten Wendemittels senkrecht zu der Abführrichtung der Materialbahn von dem ersten Wendemittel und parallel zu der genannten Ebene der Materialbahn orientiert.

In diesem Zusammenhang sieht eine wieder andere Weiterbildung der erfindungsgemäßen Wendeeinrichtung vor, dass ein für die Führung der Materialbahn wirksamer Außendurchmesser der Mantelfläche des dritten Wendemittels derart gewählt und angeordnet ist, dass der genannte Außendurchmesser einem Abstand zwischen einer Abführebene der Materialbahn von dem ersten Wendemittel und einer Zuführebene in der Materialbahn zu dem zweiten Wendemittel entspricht. Der genannte Abstand wird dabei senkrecht zu der genannten Ebene der Materialbahn bestimmt.

Die Materialbahn verlässt das erste Wendemittel in einer gedachten Ebene, welche vorliegend als "Abführebene" bezeichnet wurde. Nach der erforderlichen weiteren Umlenkung der Förderrichtung im Bereich zwischen dem ersten Wendemittel und dem zweiten Wendemittel, was gemäß der eben erläuterten Weiterbildung der erfindungsgemäßen Wendeeinrichtung durch das dritte Wendemittel bewirkt ist, erreicht die Materialbahn das zweite Wendemittel auf einer anderen gedachten Ebene, welche vorliegend als "Zuführebene" bezeichnet wurde. Vorteilhafterweise entspricht nun der wirksame effektive Außendurchmesser des dritten Wendemittels gerade dem Abstand zwischen der genannten Abführebene und der genannten Zuführebene, wodurch eine möglichst prozesssichere und platzsparende Anordnung erreichbar ist.

Eine wieder andere Weiterbildung der erfindungsgemäßen Wendeeinrichtung kann in diesem Zusammenhang vorsehen, dass das dritte Wendemittel wenigstens zwei längliche Wendemittelelemente mit zumindest abschnittweise gewölbter Mantelfläche aufweist, so dass die Materialbahn entsprechend über die Mantelflächen aller dieser Wendemittelelemente geführt ist. Die genannten Wendemittelelemente sind in einer Richtung senkrecht zu der genannten Ebene der Materialbahn beabstandet übereinander angeordnet und weisen parallele Längsachsen auf. Die Summe aus den Außendurchmessern der Wendemittelelemente und deren Zwischenabständen ergeben den genannten effektiven Außendurchmesser.

Eine andere bevorzugte Weiterbildung der erfindungsgemäßen Wendeeinrichtung sieht vor, dass wenigstens ein Wendemittel aus der das erste Wendemittel, das zweite Wendemittel und das dritte Wendemittel umfassenden Gruppe als Kreiszylinder, vorzugsweise als gerader Kreiszylinder, ausgebildet ist.

Eine wieder andere Weiterbildung der erfindungsgemäßen Wendeeinrichtung sieht in diesem Zusammenhang vor, dass zumindest das erste Wendemittel und/oder das zweite Wendemittel als Wendestange ausgebildet ist. Derartige Wendestangen sind dem Fachmann bekannt und können des weiteren zur Ausbildung eines reibungsmindernden Luftpolsters von innen pneumatisch beaufschlagbar sein. Natürlich kann auch das dritte Wendemittel als Wendestange ausgebildet sein.

Um ein störungsfreies Durchlaufen der Wendeeinrichtung für die Materialbahn zu ermöglichen, sieht eine wieder andere Weiterentwicklung der erfindungsgemäßen Wendeeinrichtung vor, dass zumindest ein Wendemittel aus der Gruppe, welche das erste Wendemittel, das zweite Wendemittel und das dritte Wendemittel umfasst, in seiner Lage bezüglich der genannten Ebene der Materialbahn bzw. bezüglich der anderen Wendemittel einstellbar ausgebildet ist. Auf diese Weise lässt sich eine sichere und verschleißarme Führung der Materialbahn innerhalb der Wendeeinrichtung sicherstellen.

Zu dem gleichen Zweck kann im Rahmen einer anderen Weiterbildung der erfindungsgemäßen Wendeeinrichtung auch vorgesehen sein, dass das erste Wendemittel und das zweite Wendemittel bezüglich ihres Relativwinkels einstellbar ausgebildet sind.

Vorteilhafterweise sind im Rahmen einer wieder anderen Weiterbildung der erfindungsgemäßen Wendeeinrichtung das erste Wendemittel und/oder das zweite Wendemittel und/oder das dritte Wendemittel an einer Tragrahmenkonstruktion angeordnet. Insbesondere im Zuge einer Ausgestaltung des dritten Wendemittels mit einer Mehrzahl von Wendemittelelementen können diese Elemente an einer gemeinsamen Tragrahmen- oder Halterrahmenkonstruktion angeordnet sein. Dies ermöglicht einen einfach zu handhabenden modularen Aufbau der Wendeeinrichtung.

In Weiterbildung dieser Idee kann vorgesehen sein, dass die Tragrahmenkonstruktion wenigstens ein erstes Rahmenteil umfasst, wobei das erste Wendemittel und das zweite Wendemittel auf unterschiedlichen Seiten dieses Rahmenteils angeordnet sein können. Wenn das Rahmenteil zudem einen Durchbruch aufweist, ist es im montierten Zustand der Wendemittel leicht möglich, den Verlauf der Materialbahn im Bereich der Wendemittel visuell zu inspizieren.

Im Zuge einer wieder anderen Weiterbildung dieser Idee kann noch vorgesehen sein, dass die Tragrahmenkonstruktion wenigstens noch ein zweites Rahmenteil umfasst, welches zweite Rahmenteil zu dem erstgenannten Rahmenteil beabstandet angeordnet ist. Dabei kann das erste Wendemittel oder das zweite Wendemittel in einem Bereich zwischen den beiden Rahmenteilen angeordnet sein, während das jeweils andere Wendemittel oberhalb oder unterhalb der genannten Rahmenteile angeordnet ist. Eine solche Ausgestaltung trägt in besonderem Maße zu einer stabilen und zugleich platzsparenden Anordnung der Wendemittel bei.

Ein anderer Aspekt der vorliegenden Erfindung richtet sich auf die Schaffung einer Materialbahnverarbeitungsmaschine gemäß Anspruch 13 mit wenigstens einer Förderrichtung für eine Materialbahn sowie mit wenigstens einer ersten Bearbeitungseinheit zum Bearbeiten der Materialbahn und mit wenigstens einer zweiten Bearbeitungseinheit zum Bearbeiten der Materialbahn.

Um die Vorteile der vorstehend detailliert beschriebenen Wendeeinrichtung im Kontext einer derartigen Materialbahnverarbeitungsmaschine nutzen zu können, schlägt die vorliegende Erfindung zusätzlich vor, eine derartige Materialbahnverarbeitungsmaschine mit einer erfindungsgemäßen Wendeeinrichtung auszurüsten, wobei die Wendeeinrichtung zwischen der ersten Bearbeitungseinheit und der zweiten Bearbeitungseinheit angeordnet ist. Auf diese Weise ist es möglich, die Materialbahn mit der ersten Bearbeitungseinheit auf ihrer einen Seite (Oberseite) und mit der zweiten Bearbeitungseinheit auf Ihre anderen Seite (Unterseite) zu bearbeiten. Da die Materialbahn durch die erfindungsgemäße Wendeeinrichtung gewendet wurde, ist es möglich, mit der ersten Bearbeitungseinheit und mit der zweiten Bearbeitungseinheit aus derselben Richtung auf die Materialbahn einzuwirken.

Eine erste Weiterbildung sieht vor, dass die gemeinsame Bearbeitungsrichtung in etwa in Richtung der Schwerebeschleunigung orientiert ist.

Des Weiteren kann vorgesehen sein, dass die erste Bearbeitungseinheit und/oder die zweite Bearbeitungseinheit dazu ausgebildet ist, wenigstens ein weiteres Material auf der Materialbahn bzw. dem Produktionsstrang zu applizieren, vorzugsweise durch Aufkleben, Aufpressen, Aufprägen, Aufsiegeln, Aufschweißen oder dergleichen. Dies kann flächig, teilflächig, lückenlos oder lückenhaft sowie überlappend geschehen.

Weitere Eigenschaften und Vorteile der vorliegenden Erfindung ergeben sich aus der nachfolgenden Beschreibung von Ausführungsbeispielen anhand der Zeichnung.
- Figur 1: zeigt perspektivisch eine erfindungsgemäße Wendeeinrichtung für eine Materialbahn;
- Figur 2: zeigt eine bevorzugte Verwendung der Wendeeinrichtung aus Figur 1 in einer Materialbahnverarbeitungsmachine;
- Figur 3: zeigt die Wendeeinrichtung gemäß Figur 1 in der Draufsicht;
- Figur 4: zeigt die Wendeeinrichtung gemäß Figur 1 in einer ersten Seitenansicht;
- Figur 5: zeigt die Wendeeinrichtung gemäß Figur 1 in einer zweiten Seitenansicht; und
- Figur 6: zeigt die Wendeeinrichtung aus Figur 1 im Schnitt entlang der Linie A-A in Figur 5.

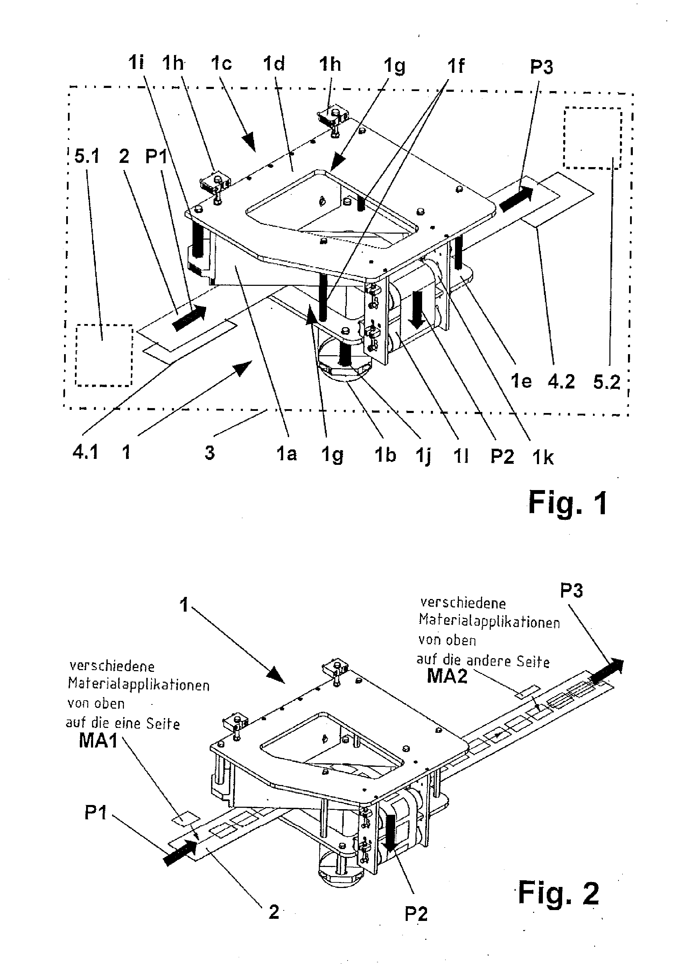

Figur 1 zeigt perspektivisch eine erfindungsgemäße Wendeeinrichtung für eine Materialbahn, welche Wendeeinrichtung in ihrer Gesamtheit mit dem Bezugszeichen 1 bezeichnet ist. Bezugszeichen 2 steht für die Materialbahn, bei welcher Materialbahn 2 es sich ohne Beschränkung der Allgemeinheit um eine Materialbahn handeln kann, welche aufgrund ihrer Zusammensetzung für die Herstellung von Hygieneartikeln, wie Damenbinden, Babywindeln, Inkontinenzwindeln, Slipeinlagen oder dergleichen ausgebildet sein kann. Zu diesem Zweck kann die Materialbahn 2 mehrlagig aufgebaut sein und dabei insbesondere auf der einen Seite ein so genanntes Topsheet aufweisen, welches in der Regel aus einem durchlässigen (Nonwoven-)Material besteht, und auf der anderen Seite ein so genanntes Backsheet, welches flüssigkeitsdicht ausgebildet ist. Zwischen den beiden vorstehend genannten Materiallagen kann ein saugfähiger Kern angeordnet sein, der beispielsweise aus gemahlener Zellulose (Flocke) besteht. Bei den weiter oben genannten Hygieneprodukten stellt das Topsheet die körperzugewandte Seite des Produkts dar, während das Backsheet die körperabgewandte Seite bildet.

Die vorliegende Erfindung ist jedoch nicht auf die Verwendung der weiter oben beschriebenen Art von Materialbahn und die Herstellung der weiter oben exemplarisch aufgezählten Produkte beschränkt, sondern kann allgemein bei Maschinen Verwendung finden, die zum Transportieren und Verarbeiten einer Materialbahn ausgebildet bzw. vorgesehen sind.

Eine solche Maschine ist in Figur 1 nur symbolisch anhand einer strichpunktierten Box 3 dargestellt. Neben der Wendeeinrichtung 1 umfasst die Materialbahnverarbeitungsmaschine 3 wenigstens noch eine Fördereinrichtung (nicht gezeigt) für die Materialbahn 2, um die Materialbahn 2 innerhalb der Materialverarbeitungsmaschine 3 zu transportieren. Als Teil dieser Fördereinrichtung sind in Figur 1 zwei so genannte Vakuumunterbänder 4.1, 4.2 dargestellt, welche einerseits zum Fördern der Materialbahn 2 in Richtung der Pfeile P1 - P3 und andererseits zum Ansaugen der Materialbahn 2 ausgebildet sind, um diese insbesondere im Zusammenhang mit bestimmten Bearbeitungsschritten definiert zu positionieren bzw. um die Materialbahn durch Hinaussaugen von Luft genauer bearbeiten zu können, weil die Materialbahn dadurch entsprechend "steifer" wird. Insbesondere beim Falten von aus der Materialbahn hergestellten Produkten kann dies von Vorteil sein. Das angesprochene "Versteifen" der Materialbahn kann dann erfolgen, wenn diese mit ihrer luftdurchlässigen Seite (Topsheet) - soweit vorhanden - zum Transportband (Vakuumunterband) 4.1, 4.2 transportiert wird. Eine weitere Bearbeitung der Materialbahn 2 kann dann auf deren Rückseite (Backsheet) erfolgen. Bestimmte Produkte, wie beispielsweise Windeln, erfordern jedoch eine andere Art der Bearbeitung und werden deshalb in der Regel mit dem Backsheet zu den Transportbändern (Vakuumunterband) 4.1, 4.2 transportiert.

Daraus ergibt sich - je nach Produktaufbau und Anwenderwunsch - die Notwendigkeit, eine Wendeeinrichtung 1 vorzusehen, um Bearbeitungsschritte an beiden Seiten der Materialbahn 2 bzw. des daraus gefertigten Produkts vornehmen zu können. Um dies zu illustrieren, sind in Figur 2 symbolisch noch eine erste Bearbeitungseinheit 5.1 und eine zweite Bearbeitungseinheit 5.2 zum Bearbeiten der Materialbahn 2 eingezeichnet. Wie sich aus der Figur 1 noch ergibt, ist die Wendeeinrichtung 1 zwischen der ersten Bearbeitungseinheit 5.1 und der zweiten Bearbeitungseinheit 5.2 angeordnet, so dass es aufgrund der Verwendung der Wendeeinrichtung 1 möglich ist, die Materialbahn beidseitig zu bearbeiten. Dies ist in Figur 2 nochmals anschaulich dargestellt, wobei hier - wie in allen Figuren - gleiche Bezugszeichen gleiche oder gleichwirkende Elemente bezeichnen.

Wie weiter oben bereits ausgeführt, sieht eine bevorzugte Ausgestaltung vor, dass die erste Bearbeitungseinheit und/oder die zweite Bearbeitungseinheit dazu ausgebildet ist, wenigstens ein weiteres Material auf der Materialbahn zu applizieren, vorzugsweise durch Aufkleben, Aufpressen, Aufprägen, Aufsiegeln, Aufschweißen oder dergleichen.

In Figur 2 bezeichnet das Bezugszeichen MA1 entsprechend eine erste Materialapplikation, die von oben auf eine erste Seite der Materialbahn 2 vor Durchlaufen der Wendeeinrichtung 1 aufgebracht wird, insbesondere durch die Bearbeitungseinheit 5.1 gemäß Figur 1. Anschließend wird die Materialbahn 2 durch die Wendeeinrichtung 1 gewendet, worauf weiter unten noch genauer eingegangen wird, so dass anschließend eine andere Materialapplikation MA2 ebenfalls von oben, das heißt in Richtung der Schwerkraft jedoch auf die andere Seite der Materialbahn 2 aufgebracht werden kann, insbesondere durch die Bearbeitungseinheit 5.2 gemäß Figur 2. Die Materialbahn 2 lässt sich also beidseitig bearbeiten, wohl jeweils nur aus einer Richtung, nämlich von oben, auf die Materialbahn 2 eingewirkt wird. Wie der Fachmann erkennt, liegt es jedoch ebenfalls im Rahmen der vorliegenden Erfindung, nur von unten auf die Materialbahn 2 einzuwirken. Ebenso gut ist bei entsprechender Führung der Materialbahn auch ein seitliches Einwirken von jeweils einer Seite möglich. Selbstverständlich ist die Erfindung nicht auf nur zwei Materialapplikationen bzw. einmaliges Wenden der Materialbahn beschränkt.

Anschließend wird die so bearbeitete Materialbahn 2 bzw. der Produktionsstrang regelmäßig gefaltet und/oder geschnitten, um einzelnen Hygieneprodukte herzustellen. Dies ist vorliegend nicht zeichnerisch dargestellt.

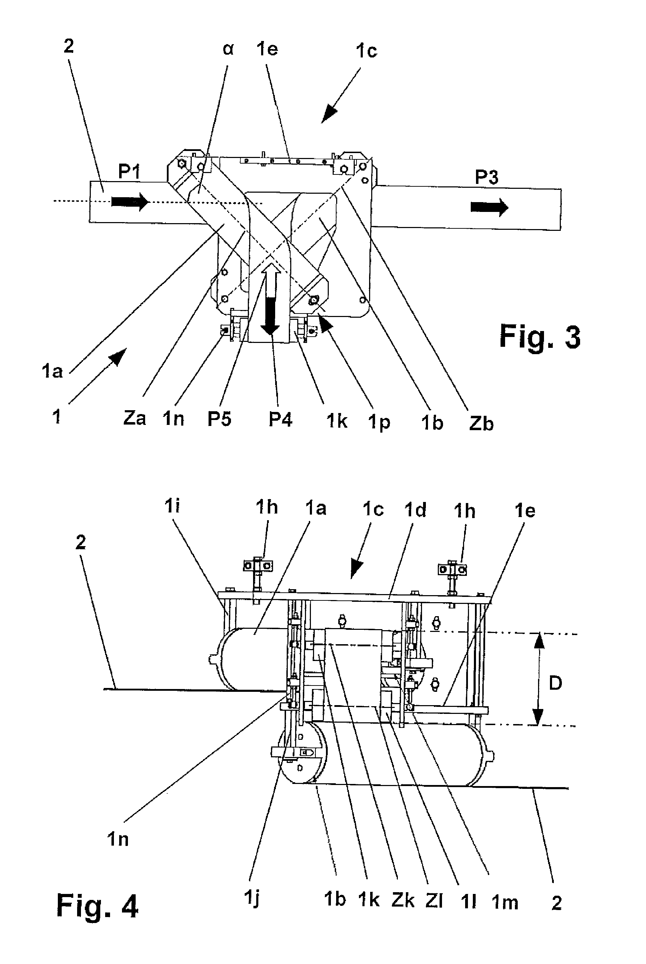

Nachfolgend wird anhand der Figuren 1 bis 6 genauer auf den konstruktiven Aufbau der Wendeeinrichtung 1 eingegangen:
Die Wendeeinrichtung 1 umfasst als Wendemittel eine erste pneumatisch beaufschlagbare Wendestange 1 a und eine zweite pneumatisch beaufschlagbare Wendestange 1b, welche relativ zueinander unter einem rechten Winkel höhenversetzt angeordnet ist, wie insbesondere der Figur 3 entnommen werden kann.
Die Wendestangen 1 a und 1b sind an bzw. in einer Rahmenkonstruktion 1 c aufgenommen, welche ein oberes Rahmenteil 1 d und ein unteres Rahmenteil 1 e umfasst, welche Rahmenteile 1d, 1e über Abstandselemente 1f miteinander verbunden sind. Die erste Wendestange 1 a ist zwischen den Rahmenteilen 1 d, 1 e angeordnet, während die zweite Wendestange 1 b außerhalb der Rahmenkonstruktion 1 c unterhalb des zweiten Rahmenteils 1e angeordnet ist. Beide Rahmenteile 1d, 1e weisen einen Durchbruch 1g auf. Bei Bezugszeichen 1h sind Befestigungselemente zum Befestigen der Wendeeinrichtung 1 an bzw. innerhalb der Materialbahnverarbeitungsmaschine 3 dargestellt. Bezugszeichen 1 i bezeichnet Befestigungselemente zum Befestigen der ersten Wendestange 1 a an dem ersten Rahmenteil 1d, und Bezugszeichen 1j bezeichnet entsprechende Befestigungselemente für die zweite Wendestange 1b an dem zweiten Rahmenteil 1e. Figur 3 zeigt eine Draufsicht auf die Wendeeinrichtung 1 gemäß Figur 1 und Figur 2 ohne das obere Rahmenteil 1d.

Die Wendestangen 1 a und 1b weisen in an sich bekannter Weise eine äußere Mantelfläche nach Art eines glatten Kreiszylinders mit entsprechender Zylinder- bzw. Längsachse auf, auf welcher die Materialbahn 2 geführt ist. Ebenso sind die Wendestangen 1 a und 1 b in an sich bekannter Weise von innen pneumatisch beaufschlagt, so dass sich über ihrer Außenfläche ein Luftpolster bildet, auf welchem die Materialbahn 2 geführt ist, um Reibungseffekte zu minimieren.

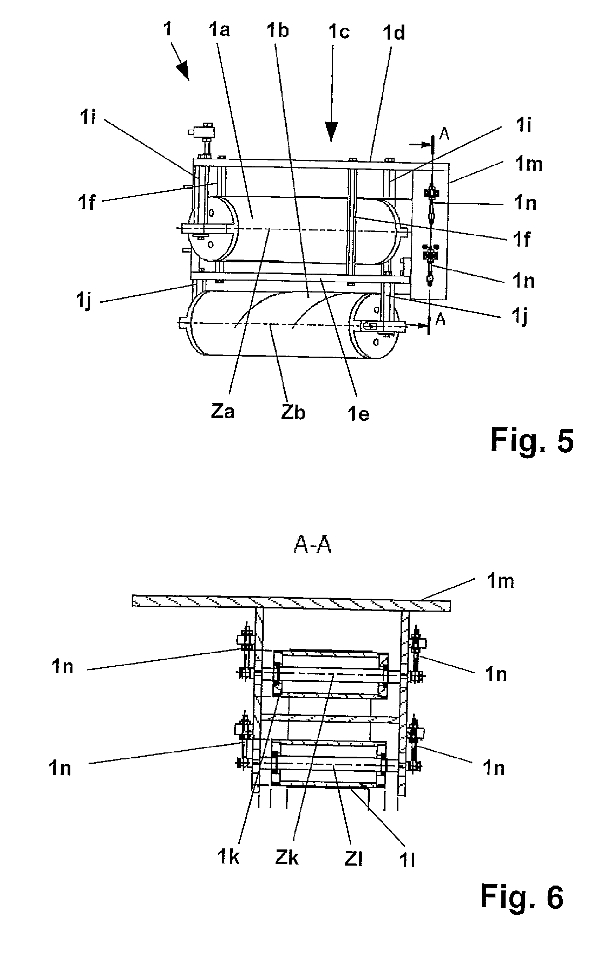

Bezogen auf die durch den Pfeil P1 symbolisierte Förderrichtung der Materialbahn 2 vor der ersten Wendestange 1 a ist Letztere unter einem Winkel α = 45° zu der genannten Förderrichtung P1 orientiert, was ebenfalls in Figur 3 besonders gut erkennbar ist. Die Zylinderachse der ersten Wendestange 1a und die Zylinderachse der zweiten Wendestange 1 b sind jeweils parallel zu einer Ebene in der Materialbahn 2 vor der Umlenkung durch die erste Wendestange 1 a orientiert, wie sich insbesondere in der Figur 5 gut entnehmen lässt. Hier ist die genannte Ebene der Materialbahn horizontal und senkrecht zur Zeichenebene orientiert.

Die genannten Längs- bzw. Zylinderachsen der Wendemittels sind in Figur 5 gestrichelt eingezeichnet und mit Za für die Zylinderachse der Wendestange 1 a bzw. mit Zb für die Zylinderachse der Wendestange 1 b bezeichnet.

Insbesondere in den Figuren 1, 2, 3, 4 und 6 sind außerdem weiterhin noch weitere Wendemittel in Form von zylinderförmigen Wendemittelelementen (Stangen oder Rollen) dargestellt, die in den genannten Figuren mit 1k bzw. 1l bezeichnet und senkrecht übereinander beabstandet angeordnet sind, wobei ihre Zylinderachsen Zk, Zl parallel orientiert sind. Die vorstehend genannten Wendemittelelemente 1k, 1l sind derart angeordnet und ausgebildet, dass die Materialbahn 2 zwischen der ersten Wendestange 1 a und der zweiten Wendestange 1b so verläuft, dass eine Zuführrichtung der Materialbahn 2 zu der zweiten Wendestange 1 b antiparallel zu einer Abführrichtung der Materialbahn von der ersten Wendestange 1 a ausgebildet ist. Dies ist insbesondere der Abbildung in Figur 3 gut zu entnehmen. Dort ist die Abführrichtung der Materialbahn von der ersten Wendestange 1 a durch einen Pfeil P4 symbolisiert, während die Zuführrichtung der Materialbahn zu der zweiten Wendestange 1b, welche gemäß der Darstellung in Figur 3 in einer tiefer liegenden Ebene erfolgt, durch einen Pfeil P5 symbolisiert ist. Für die entsprechende Umlenkung sorgen die Wendemittelelemente 1 k, 1l (vgl. Figur 1), von denen in Figur 3 aus darstellungstechnischen Gründen nur das obere Wendemittelelement 1 k erkennbar ist. Anschließend sorgt die zweite Wendestange 1 b nach einer weiteren Umlenkung der Bewegungsrichtung der Materialbahn 2 um 90° dafür, dass die Materialbahn 2 die Wendeeinrichtung 1 parallel zur ursprünglichen Zuführrichtung P1 in Richtung des Pfeils P3 gewendet wieder verlässt, so dass die vormalige Oberseite der Materialbahn 2 nunmehr unten liegt, und umgekehrt.

Wie der Fachmann erkennt, könnte anstelle der übereinander angeordneten Wendemittelelemente 1 k, 1l auch ein einziges Wendemittelelement mit entsprechend vergrößerten Abmessungen zum Einsatz kommen. Auch eine Umkehr der Laufrichtung der Materialbahn 2 ist problemlos realisierbar.

Was die relative Dimensionierung der Wendemittel (Wendestangen 1a, 1 b und Wendemittelelemente 1k, 1l) anbelangt, sei nachfolgend insbesondere auf die Darstellung in Figur 4 Bezug genommen. Wie man der Figur 4 leicht entnimmt, entspricht der zur Führung der Materialbahn 2 effektiv wirksame Außendurchmesser der kombinierten Wendemitteleiemente 1 k, 11, welcher Außendurchmesser in Figur 4 mit dem Bezugszeichen D bezeichnet ist, im Wesentlichen gerade einem Abstand zwischen einer Abführebene der Materialbahn 2 von der ersten Wendestange 1a und einer Zuführebene der Materialbahn 2 zu der zweiten Wendestange 1b. Gemäß der Darstellung in Figur 4 wird die Materialbahn 2 von der Oberseite der Zylinderstange 1 a ab - und anschließend nach Umlenkung durch die Wendemittelelemente 1k, 1l der Oberseite der Wendestange 1 b zugeführt. Der genannte Außendurchmesser D entspricht also gerade dem Abstand zwischen den genannten Oberseiten der Wendestangen 1 a, 1 b bzw. dem Abstand zwischen zwei gedachten parallelen Ebenen (in Figur 4 nicht dargestellt), welche die genannten Wendestangenoberseiten enthalten.

Darüber hinaus ist der Figur 4 noch die relative Lage der Zylinderachsen der Wendemittelelemente 1k, 1l zu entnehmen, welche dort mit dem Bezugszeichen Zk bzw. Zl bezeichnet sind, wie bereits erwähnt wurde.

Zumindest die Wendemittelelemente 1 k, 1l sind an einer eigenen Halterahmenkonstruktion 1 m gelagert, welche mit der Rahmenkonstruktion 1 c bzw. den Rahmenteilen 1 e, 1 d verbunden ist.

Wie den Figuren weiterhin zu entnehmen ist, sind die Wendemittelelemente 1k, 1l innerhalb des Halterahmens 1m höhenverstellbar gelagert, wozu entsprechende Verstelleinrichtungen 1 n vorgesehen sind, die aus Gründen der Übersichtlichkeit nicht in allen Figuren explizit bezeichnet sind.

Vorteilhafterweise weist die Wendeeinrichtung 1 eine entsprechende Verstellbarkeit auch für die Wendestangen 1 a, 1 b auf. Des Weiteren kann auch eine Verstellbarkeit der Wendestangen 1 a, 1 b bezüglich ihres relativen Winkels (rechter Winkel gemäß Figur 3) vorgesehen sein, beispielsweise indem an den Befestigungen der Wendestangen 1 a, 1b bezüglich der Rahmenkonstruktion 1c Langlöcher oder dergleichen vorgesehen sind, welche die angesprochene Einstellbarkeit des Relativwinkels ermöglichen. Dies ist in Figur 3 für die Wendestange 1a bei Bezugszeichen 1 p angedeutet.

Wie der Figur 3 noch zu entnehmen ist, dient der bereits erwähnte Durchbruch 1g insbesondere zum Inspizieren der Materialbahnführung im Umlenkbereich der Wendestangen 1 a, 1 b. Die Materialbahn selbst wird mittels der Wendemittelelemente 1 k, 1l seitlich an dem Rahmenteil 1e vorbeigeführt, wenn sie von der höheren Führung im Bereich der ersten Wendestange 1 a zu der tieferen Führung im Bereich der zweiten Wendestange 1 b wechselt.

## Patentansprüche

1. Verfahren zum Bearbeiten einer Materialbahn (2), insbesondere zur Herstellung von Hygieneprodukten, wie Einlagen, Windeln oder dergleichen, bei dem an oder auf der Materialbahn weitere Materialien angebracht oder aufgebracht werden, vorzugsweise aufgeklebt, aufgepresst, aufgeriegelt oder dergleichen,
**dadurch gekennzeichnet, dass**
die Materialbahn (2)
a) mittels wenigstens einer ersten Bearbeitungseinheit (5.1) mit einer ersten Bearbeitungsrichtung, vorzugsweise von oben in Richtung der Schwerebeschleunigung, bearbeitet wird, wobei eine erste Materialapplikation (MA1) erfolgt.
b) durch wenigstens eine Wendeeinrichtung (1) geführt wird, um die Materialbahn (2) in der Wendeeinrichtung (1) im Wesentlichen um 180° zu wenden, ohne eine Förderrichtung der Materialbahn (2) zu verändern;
c) mittels wenigstens einer zweiten Bearbeitungseinheit (5.2) mit einer zweiten Bearbeitungsrichtung, vorzugsweise von oben in Richtung der Schwerebeschleunigung, bearbeitet wird, wobei eine zweite Materialappiikation (MA2) erfolgt;
wobei die erste Bearbeitungsrichtung und die zweite Bearbeitungsrichtung im Wesentlichen gleich orientiert sind.

2. Verfahren nach Anspruch 1,
**dadurch gekennzeichnet, dass**
die erste Bearbeitung und die zweite Bearbeitung im Wesentlichen von oben nach unten in Richtung der Schwerebeschleunigung erfolgen.

3. Verfahren nach Anspruch 1 oder 2,
**dadurch gekennzeichnet, dass**
die Materialbahn (2) mehrlagig aufgebaut ist, mit einem Topsheet aus einem für Fluid durchlässigen Material und mit einem fluiddichten Backsheet, zwischen denen vorzugweise ein saugfähiger Kern angeordnet ist, und dass das Verfahren zur Herstellung von Hygieneprodukten eingesetzt wird, vorzugsweise für Binden, Windeln oder Einlagen.

4. Verfahren nach Anspruch 1 bis 3,
**dadurch gekennzeichnet, dass**
in Schritt b) eine Wendeeinrichtung (1) gemäß einem der nachfolgenden Ansprüche zum Wenden der Materialbahn (2) verwendet wird.

5. Wendeeinrichtung (1) für eine Materialbahn (2), zur Durchführung zumindest von Schritt b) des Verfahrens gemäß einem der Ansprüche 1 bis 4, mit wenigstens einem ersten länglichen Wendemittel (1a) mit zumindest abschnittweise gewölbter Mantelfläche, über dessen Mantelfläche die Materialbahn (2) geführt ist, und mit wenigstens einem in Förderrichtung (P1, P2) der Materialbahn (2) auf das erste Wendemittel (1a) folgenden zweiten länglichen Wendemittel (1b) mit zumindest abschnittweise gewölbter Mantelfläche, über dessen Mantelfläche die Materialbahn (2) geführt ist, wobei eine Längsachse (7a) des ersten Wendemittels (1a) unter einem Winkel (α) von 45° zu einer Förderrichtung (P1) der Materialbahn (2) vor einer Umlenkung durch das erste Wendemittel (1a) orientiert ist, wobei die Längsachse (7a) des ersten Wendemittels (1a) und eine Längsachse (7b) des zweiten Wendemittels (1b) unter einem rechten Winkel zueinander und jeweils parallel zu einer Ebene der Materialbahn (2) vor der Umlenkung durch das erste Wendemittel (1a) orientiert sind, und wobei die Materialbahn (2) zwischen dem ersten Wendemittel (1a) und dem zweiten Wendemittel (1 b) derart verläuft, dass eine Zuführrichtung (P5) der Materialbahn (2) zu dem zweiten Wendemittel (1 b) antiparallel zu einer Abführrichtung (P4) der Materialbahn (2) von dem ersten Wendemittel (1a) ausgebildet ist.

6. Wendeeinrichtung (1) nach Anspruch 5,
**dadurch gekennzeichnet, dass**
das erste Wendemittel (1a) und das zweite Wendemittel (1 b) einander in einer Richtung senkrecht zu der genannten Ebene der Materialbahn (2) zumindest teilweise überlappen.

7. Wendeeinrichtung (1) nach Anspruch 5 oder 6,
**dadurch gekennzeichnet, dass**
in Förderrichtung der Materialbahn (2) zwischen dem ersten Wendemittel (1a) und dem zweiten Wendemittel (1 b) wenigstens ein drittes längliches Wendemittel (1k, 1l) mit zumindest abschnittweise gewölbter Mantelfläche angeordnet ist, über dessen Mantelfläche die Materialbahn (2) geführt ist, wobei eine Längsachse (7k, 7l) des dritten Wendemittels (1k, 1l) senkrecht zu der Abführrichtung (P4) der Materialbahn von dem ersten Wendemittel (1a) und parallel zu der genannten Ebene der Materialbahn (2) orientiert ist.

8. Wendeeinrichtung (1) nach Anspruch 7,
**dadurch gekennzeichnet, dass**
ein für die Führung der Materialbahn (2) wirksamer Außendurchmesser (D) der Mantelfläche des dritten Wendemittels (1k, 1l) derart gewählt und angeordnet ist, dass der genannte Außendurchmesser (D) einem Abstand zwischen einer Abführebene der Materialbahn (2) von dem ersten Wendemittel (1a) und einer Zuführebene der Materialbahn (2) zu dem zweiten Wendemittel (1 b) entspricht, wobei der Abstand senkrecht zu der genannten Ebene der Materialbahn (2) bestimmt ist, und dass vorzugsweise das dritte Wendemittel (1k, 1l) wenigstens zwei längliche Wendemittelelemente (1k, 1l) aufweist, die in einer Richtung senkrecht zu der genannten Ebene der Materialbahn (2) beabstandet übereinander und mit parallelen Längsachsen (7k, 7l) angeordnet sind.

9. Wendeeinrichtung (1) nach mindestens einem der Ansprüche 5 bis 8,
**dadurch gekennzeichnet, dass**
das erste Wendemittel (1 a) und/oder das zweite Wendemittel (1 b) und/oder das dritte Wendemittel (1k, 1l) gemäß Anspruch 7 als Wendestange ausgebildet ist, vorzugsweise als zur Ausbildung eines Luftpolsters von innen mit Druckluft beaufschlagbare Wendestange.

10. Wendeeinrichtung (1) nach mindestens einem der Ansprüche 5 bis 9,
**dadurch gekennzeichnet, dass**
das erste Wendemittel (1a) und/oder das zweite Wendemittel (1 b) und/oder das dritte Wendemittel (1k, 1l) gemäß Anspruch 7 in seiner Lage bezüglich der genannten Ebene der Materialbahn (2) einstellbar ausgebildet ist.

11. Wendeeinrichtung (1) nach mindestens einem der Ansprüche 5 bis 10,
**dadurch gekennzeichnet, dass**
das erste Wendemütel (1 a) und/oder das zweite Wendemittel (1 b) und/oder das dritte Wendemittel (1k, 1l) gemäß Anspruch 7 an einer Tragrahmenkonstruktion (1c, 1 m) angeordnet sind, wobei vorzugsweise die Tragrahmenkonstruktion (1c) wenigstens ein erstes Rahmenteil (1d) umfasst, wobei das erste Wendemittel (1b) und das zweite Wendemittel auf unterschiedlichen Seiten des ersten Rahmenteils (1e) angeordnet sind, welches erste Rahmenteil (1c) höchst vorzugsweise einen Durchbruch (1g) zum Inspizieren der Materialbahn (2) aufweist.

12. Wendeeinrichtung (1) nach Anspruch 11,
**dadurch gekennzeichnet, dass**
die Tragrahmenkonstruktion (1c) wenigstens noch ein zweites Rahmenteil (1d) umfasst, welches zweite Rahmenteil (1d) zu dem ersten Rahmenteil (1e) beabstandet angeordnet ist, wobei das erste Wendemittel (1a) oder das zweite Wendemittel in einem Bereich zwischen dem ersten Rahmenteil (1e) und dem zweiten Rahmenteil (1d) angeordnet ist.

13. Materialbahnverarbeitungsmaschine (3) mit wenigstens einer Fördereinrichtung (4.1, 4.2) für eine Materialbahn (2), mit wenigstens einer ersten Bearbeitungseinheit (5.1) zum Bearbeiten der Materialbahn (2) und mit wenigstens einer zweiten Bearbeitungseinheit (5.2) zum Bearbeiten der Materialbahn (2), vorzugsweise zur Herstellung von Hygieneprodukten, **gekennzeichnet durch**
eine Wendeeinrichtung (1) nach einem der Ansprüche 5 bis 12, welche Wendeeinrichtung (1) zwischen der ersten Bearbeitungseinheit (5.1) und der zweiten Bearbeitungseinheit (5.2) angeordnet ist, wobei die erste Bearbeitungseinheit (5.1) und die zweite Bearbeitungseinheit (5.2) dazu ausgebildet und angeordnet sind, die Materialbahn (2) in einer gemeinsamen Bearbeitungsrichtung zu bearbeiten, vorzugsweise von oben in Richtung der Schwerkraft, höchst vorzugsweise in Form jeweils einer Materialapplikation (MA1, MA2) auf oder an der Materialbahn (2).

14. Materialbahnverarbeitungsmaschine (3) nach Anspruch 13,
**dadurch gekennzeichnet, dass**
die gemeinsame Bearbeitungsrichtung in etwa in Richtung der Schwerebeschleunigung orientiert ist.

15. Materialbahnverarbeitungsmaschine (3) nach Anspruch 13 oder 14,
**dadurch gekennzeichnet, dass**
die erste Bearbeitungseinheit (5.1) und/oder die zweite Bearbeitungseinheit (5.2) dazu ausgebildet ist, wenigstens ein weiteres Material auf der Materialbahn (2) zu applizieren, vorzugsweise durch Aufkleben, Aufpressen, Aufprägen, Aufsiegeln, Aufschweißen oder dergleichen.
